Europäisches Patentamt

European Patent Office

Office européen des brevets

(19)

(11) Numéro de publication: **0 185 042**
**B1**

(12) ## FASCICULE DE BREVET EUROPEEN

(45) Date de publication du fascicule du brevet:
**08.11.89**

(51) Int. Cl.⁴: **A 61 K 39/02**

(21) Numéro de dépôt: **85902582.7**

(22) Date de dépôt: **14.06.85**

(86) Numéro de dépôt international:
**PCT/FR 85/00152**

(87) Numéro de publication internationale:
**WO 86/00019 (03.01.86 Gazette 86/1)**

(54) VACCIN CONTRE LES MALADIES DUES A DES MICROORGANISMES TELS QUE DES MYCOPLASMES, SA PREPARATION ET MEMBRANES DE MICROORGANISMES EN TANT QUE PRINCIPE ACTIF.

(30) Priorité: **15.06.84 FR 8409422**

(43) Date de publication de la demande:
**25.06.86 Bulletin 86/26**

(45) Mention de la délivrance du brevet:
**08.11.89 Bulletin 89/45**

(84) Etats contractants désignés:
**BE CH DE FR GB IT LI LU NL SE**

(56) Documents cités:
**FR-A- 2 201 878**

**Biological Abstracts, vol. 72, 1981, Philadelphia (US). L.C. Lloyd et al.: "The pathological and serological response induced in pigs by parenteral inoculation of Mycoplasma hyopneumoniae" see abstract 38112, & J. Comp. Pathol. 91(1), 77-74, 1981**

(73) Titulaire: **CENTRE NATIONAL DE LA RECHERCHE SCIENTIFIQUE (CNRS), 15, Quai Anatole France, F-75007 Paris (FR)**
Titulaire: **DEPARTEMENT DES COTES DU NORD, Collectivité territoriale Place du Général de Gaulle, F-22022 Saint-Brieuc (FR)**

(72) Inventeur: **KOBISCH, Marylène, Le Clos de la Fontaine, F-22960 Pledran (FR)**
Inventeur: **QUILLIEN, Laurence, 18, square du Docteur Alphonse Guérin, F-35100 Rennes (FR)**
Inventeur: **WROBLEWSKI, Henri, 20, rue Emile Bernard, F-35000 Rennes (FR)**

(74) Mandataire: **Ayache, Monique et al, CABINET AYACHE 3, côte de la Jonchère B.P. 24, F-92502 Rueil-Malmaison Cedex (FR)**

## Description

L'invention a pour objet un vaccin contre les maladies animales provoquées par des microorganismes ayant pour enveloppe la plus externe une membrane, et plus particulièrement contre les maladies provoquées par les mycoplasmes, ainsi qu'un procédé pour sa préparation.

Les maladies provoquées par les microorganismes ayant pour enveloppe la plus externe une membrane, tels que les bactéries gram négatives et les mycoplasmes sont très répandues, en particulier dans les élevages d'animaux domestiques où elles sont responsables d'un manque à gagner important. Il en est particulièrement ainsi dans les élevages intensifs de porcins où la pneumonie enzootique du porc, provoquée par Mycoplasma hyopneumoniae est très fréquente.

Dans un article récent consacré à l'élevage moderne du porc, W. POND [Pour la Science, 66, p. 60–68 (1983)] décrit la place tenue actuellement dans le monde par ce type d'élevage. Le porc fournit notamment environ 25% de l'énergie et 9% des protéines d'origine animale. En élevage, le rendement des porcins est supérieur à celui des bovins, des ovins et des volailles.

De plus, la viande de porc possède d'excellentes qualités nutritives et est appréciée des consommateurs. Il n'est donc pas surprenant que l'élevage de porcins occupe une place non négligeable dans l'économie de nombreux pays d'Amérique (Nord et Sud), d'Europe, d'Asie et d'Océanie. Ceci concerne aussi bien des pays fortement industrialisés que des pays qui le sont beaucoup moins. En France, notamment en Bretagne, l'élevage des porcins constitue une composante essentielle de l'économie agricole.

Les techniques d'élevage des porcs ont beaucoup progressé tant au niveau des rendements obtenus, qu'au niveau des conditions d'élevage, les conditions d'hygiène en particulier. Ces progrès ne permettent pas, néanmoins, de mettre les élevages à l'abri d'un certain nombre de maladies dont les mycoplasmoses. Parmi les maladies qui affectent l'appareil respiratoire du porc, la pneumonie est très commune, en particulier dans le contexte de l'élevage intensif. Une enquête épidémiologique de trois ans, menée en Bretagne, a permis de détecter Mycoplasma hyopneumoniae dans 15 à 42% des lésions pulmonaires chez des porcelets âgés de 8 à 12 semaines. D'autre part, des anticorps anti M. hyopneumoniae ont été détectés par hémagglutination indirecte dans 80% des troupeaux. [M. Kobisch et Coll., Journées de Recherche Porcine en France (1977) p. 161–164, M. Kobisch et Coll., Rec. Méd. Vét., 154, (1978) p. 847–852 et J.P. Tillon, Journées de Recherche Porcine en France, (1980), p. 361–380]. Le rôle étiologique primaire de M. hyopneumoniae dans la pneumonie enzootique du porc a été démontré par R.F.W. Goodwin et Coll., British Vet. 77 (1973), p. 456–464 et revu par W.P. Switzer et Coll. dans Disease of Swine, (1975), 4e éd., Dunne and Leman Eds., Iowa University Press.

Les maladies respiratoires sont responsables de la baisse significative du rendement de nombreux élevages porcins. S'il est possible de lutter contres les bactéries au moyen d'antibiotiques, les mycoplasmes posent un problème spécifique, lié à leur simplicité structurale. Dépourvus de paroi, ces microorganismes sont totalement insensibles à tout antibiotique dont la cible est précisément la paroi, tels que par exemple pénicilline, céphalosporine, et bacitracine. D'autre part, la membrane plasmique des mycoplasmes serait imperméable à un certain nombre d'antibiotiques dont la cible est intracellulaire [S. Razin, Microbiol. Rev. (1978), p. 414–470].

Il résulte de ceci que la vaccination pourrait constituer un mode efficace et rentable de lutte contre les mycoplasmes.

Il n'existe pas actuellement sur le marché de vaccin contre les maladies provoquées par les mycoplasmes. Certes, on a déjà proposé des vaccins contre ces maladies, tels que des vaccins vivants utilisant des souches atténuées (brevet US 3 534 136), ou des vaccins renfermant des cellules complètes de mycoplasmes tuées par la chaleur, un agent tel que le formol, le salicytate d'éthylmercure sodium ou la β-propiolactone, ou par sonication (demande de brevet FR 2 201 878). Toutefois, aucun de ces vaccins ne s'est, pour diverses raisons, révélé satisfaisant et n'est utilisé couramment.

Selon l'invention, on a établi que la membrane plasmique d'un microorganisme ayant pour enveloppe la plus externe une membrane, en particulier d'un mycoplasme, peut constituer, lorsqu'elle est séparée du reste de la cellule, le principe actif particulièrement efficace d'un vaccin contre les maladies provoquées par le microorganisme concerné.

L'invention a donc pour objet un vaccin destiné à protéger contre les maladies provoquées par un microorganisme ayant pour enveloppe la plus externe une membrane, en particulier un mycoplasme, caractérisé en ce qu'il contient, en tant que principe actif, des membranes plasmiques du microorganisme concerné, débarrassées, au moins dans une large mesure, des autres constituants dudit microorganisme.

Les microorganismes concernés par l'invention sont notamment les bactéries gram négatives et surtout les mycoplasmes. Parmi ces derniers on peut citer:

– en pathologie aviaire: Mycoplasma gallisepticum, Mycoplasma synoviae et Mycoplasma meleagridis;

– en pathologie bovine: Mycoplasma mycoïdes, Mycoplasma bovis, Mycoplasma bovirhinis et Mycoplasma bovigenitalium;

– en pathologie ovine et caprine: Mycoplasma agalactiae, Mycoplasma ovipneumoniae, Mycoplasma capricolum, et Mycoplasma mycoïdes subspecies capri;

– en pathologie du cheval: Mycoplasma equigenitalium, Mycoplasma equirhinis, Acholeplasma heppikon et Acholesplasma equiletale; et

– en pathologie porcine: Mycoplasma hyopneumoniae et Mycoplasma hyorhinis.

Selon un mode préféré de réalisation, le vaccin selon l'invention se présente sous forme d'une suspension de membranes dans une solution saline dite physiologique, c'est-à-dire une solution de NaCl à 8,5 g/l, en présence d'un adjuvant qui assure un relargage progressif de l'immunogène.

Selon un mode tout particulièrement préféré de réalisation, l'adjuvant est constitué par de l'hydroxyde d'aluminium, de l'agarose ou des anticorps anti-membrane du microorganisme concerné.

D'autres adjuvants, en particulier huileux, peuvent être utilisés; il ne sont toutefois pas préférés.

La concentration en protéines provenant des membranes dans le vaccin est de préférence située dans la gamme de 2 à 3 g/l; elle est avantageusement de 2,5 g/l.

La concentration en hydroxyde d'aluminium est de préférence dans la gamme de 12 à 13 g/l, selon la concentration en protéines; elles est avantageusement de 12,5 g/l, pour une concentration en protéines de 2,5 g/l.

L'agarose est en général utilisé sous forme de gel très dilué, de préférence à une concentration comprise dans la gamme de 1,5 à 2 g/l selon la concentration en protéines; elle est avantageusement de 1,7 g/l pour une concentration en protéines de 2,5 g/l.

Selon un mode tout particulièrement préféré de réalisation, l'invention a pour objet un vaccin destiné à protéger les porcins contre la pneumonie enzootique, caractérisé en ce qu'il est essentiellement constitué par une suspension de membranes de Mycoplasma hyopneumoniae débarrassées, au moins dans une large mesure, des autres constituants du microorganisme, correspondant à un poids de protéines de 2 à 3 g/l, dans une solution aqueuse de NaCl à 8,5 g/l en présence de 12 à 13 g/l d'hydroxyde d'aluminium en tant qu'adjuvant.

Selon un autre mode tout particulièrement préféré de réalisation, l'invention a pour objet un vaccin destiné à protéger les porcins contre la pneumonie enzootique, caractérisé en ce qu'il est essentiellement constitué par une suspension de membranes de Mycoplasma hyopneumoniae débarrassées, au moins dans une large mesure, des autres constituants du microorganisme, correspondant à un poids de protéines de 2 à 3 g/l, dans une solution aqueuse de NaCl à 8,5 g/l en présence de 1,5 à 2 g/l d'agarose en tant qu'adjuvant.

L'invention a en outre pour objet un procédé pour la préparation du vaccin décrit plus haut, caractérisé en ce qu'il comprend essentiellement les étapes consistant à:

a) cultiver le microorganisme contre lequel on désire préparer un vaccin jusqu'à la fin de la phase exponentielle de croissance;

b) récolter les cellules par centrifugation;

c) lyser les cellules récoltées par les ultrasons, à 20 à 28 kHz, en plusieurs étapes, pendant 2 à 5 minutes au total;

d) sédimenter les membranes par centrifugation, et

e) les introduires dans une forme utilisable pour la vaccination.

La culture des cellules est avantageusement effectuée à 37 °C sur un milieu liquide tel que le milieu de FRIIS. [N.F. Friis, Nord. Vet. Med., 27, (1975), p. 337–339]

Les cellules centrifugées (à environ 10 000 g) sont lavées avec un tampon présentant un pH légèrement basique, par exemple du tampon phosphate de sodium 0,1M, pH 7,5, contenant du NaCl à 8,5 g/l.

Les cellules sont ensuite dispersées, par exemple au moyen d'un cylindre de Potter, de manière à obtenir une suspension dense dans du tampon légèrement basique, par exemple du tampon Tris-HCl 0,1 M, pH 8,0. La lyse par les ultrasons se fait en plusieurs étapes, avantageusement à des fréquences croissantes, dans la gamme indiquée plus haut, au voisinage de 0 °C.

Après centrifugation poussée (à environ 40 000 g, pendant environ 1 h) du lysat, les membranes sédimentées sont lavées soigneusement (plusieurs fois avec du tampon, par exemple du tampon Tris-HC1 0,1 M, pH 8,0) et dispersées puis centrifugées de nouveau plusieurs fois.

Les membranes ainsi recueillies sont prêtes à être utilisées pour préparer le vaccin. Elles peuvent être conservées à −70 °C sous forme d'une suspension concentrée dans une solution aqueuse de NaCl à 8,5 g/l, par exemple à 20 mg de protéines/ml, ou sous forme lyophilisée.

L'invention a donc en outre pour objet en tant que produits industriels nouveaux:

– une suspension concentrée dans une solution aqueuse de NaCl à 8,5 g/l de membranes d'un microorganisme ayant pour enveloppe la plus externe une membrane, en particulier d'un mycoplasme, débarrassées, au moins dans une large mesure, des autres constituants du microorganisme, pour l'utilisation en tant que principe actif d'un vaccin; et

– des membranes d'un microorganisme ayant pour enveloppe la plus externe une membrane en particulier d'un mycoplasme, débarrassées, au moins dans une large mesure, des autres constituants du microorganisme, sous forme lyophilisée, pour l'utilisation en tant que principe actif d'un vaccin.

Le vaccin selon l'invention qui ne contient pas de cellules vivantes est dépourvu de toxicité et ne présente donc pas, pour l'animal traité, les risques des vaccins vivants utilisant des souches atténuées, tout en étant plus efficace et d'un emploi plus aisé que les vaccins utilisant des cellules simplement tuées, sans séparation de la membrane plasmique, tels que ceux décrits dans la demande de brevet FR 2 201 878.

Ainsi, contrairement à ce qu'on pouvait attendre, les vaccins obtenus selon l'invention sont actifs même lorsqu'ils sont administrés par voie sous-cutanée ou intramusculaire, alors que les vaccins décrits dans la demande de brevet FR 2 201 878 n'exercent un effet préventif sur l'animal traité que lorsqu'ils sont inoculés dans la trachée par injection ou pulvérisation. De plus, les vaccins obtenus selon l'invention, lorsqu'ils sont inoculés à une femelle gestante, protègent également de façon efficace la portée à naître, ce qui représente un avantage très important pour la salubrité des élevages industriels.

Ainsi, il est possible de protéger efficacement une portée de porcelets contre les maladies provoquées par Mycoplasma hyopneumoniae en injectant à la truie gestante, par voie sous-cutanée ou intramusculaire, un inoculum tel que défini ci-dessous, 7 à 8 semaines avant la mise-bas, puis de nouveau 2 semaines avant celle-ci.

Selon un mode tout particulièrement préféré de réalisation, l'invention a pour objet un tel inoculum, ou dose unitaire, qui est caractérisé en ce qu'il est essentiellement constitué par une suspension de membranes de Mycoplasma hyopneumoniae débarrassées, au moins dans une large mesure, des autres constituants du microorganisme, et correspondant à un poids de protéines de 4 à 6 mg, de préférence de 5 mg, dans 2 ml d'une solution aqueuse de NaCl à 0,85%, en présence de 1,20 à 1,30%, de préférence 1,25%, d'hydroxyde d'aluminium ou de 0,15 à 0,20%, de préférence 0,17%, d'agarose, ou encore d'une quantité saturante pour les membranes d'anticorps de porc antimembrane de Mycoplasma hyopneumoniae.

L'invention sera mieux comprise à l'aide de la description donnée ci-dessous, à titre purement illustratif, d'un exemple de mise en œuvre de l'invention et des résultats qu'elle permet d'obtenir.

I – Obtention de vaccins anti-Mycoplasma hyopneumoniae

1) Culture du microorganisme.

Mycoplasma hyopneumoniae [souche BO 14: M.Kobisch et coll, Rec. Méd. Vét. 152 (1976), p. 817–827] est cultivée à 37 °C sur le milieu liquide décrit par FRIIS. Les cellules sont récoltées en fin de phase exponentielle de croissance par centrifugation (10 000 x g, 15 min, à 4 °C) et lavées deux foix avec du tampon phosphate de sodium 0,1 M, pH 7,5 contenant du NaCl à 8,5 g/l.

2) Isolement des membranes

Les cellules sont dispersées au moyen d'un cylindre de Potter de manière à obtenir une suspension dense dans du tampon Tris-HCl 0,1 M, pH 8,0. Elles sont ensuite lysées par les ultrasons: 2 fois 1 minute à 20kHz et 1 fois une minute à 28 kHz. L'opération est réalisée à 0 °C en laissant reposer le matériel une minute entre deux cycles consécutifs.

Le lysat cellulaire est centrifugé pendant 1 heure à 40 000 x g, à 4 °C, de manière à sédimenter les membranes. Celles-ci sont lavées quatre fois avec du tampon Tris-HCl 0,1 M, pH 8,0 par dispersions et centrifugations successives.

Le matériel obtenu est stocké à –70 °C, soit sous forme d'une suspension concentrée (20 mg de protéines/ml), soit sous forme lyophilisée.

3) Préparation des vaccins

Les vaccins sont constitués par des membranes de M. hyopneumoniae obtenues comme décrit ci-dessus, en suspension dans une solution de NaCl à 8,5 g/l. La concentration en protéines est de 2,5 mg/ml. Les adjuvants utilisés sont: hydroxyde d'aluminium à 1,25% (vaccin I), agarose à 0,17% (vaccin II) et anticorps de porc antimembrane de M.hyopneumoniae qui précipitent les membranes (vaccin III).

II – Utilisation des vaccins

Les vaccins dont la préparation est décrite ci-dessus ont été utilisés pour vacciner des truies gestantes nullipares provenant de la porcherie protégée de la Station de Pathologie Porcine de Ploufragan (Côtes du Nord, France). Le troupeau de cette porcherie est constitué d'animaux obtenus par hystérectomie et maintenus à l'abri des contaminants. Les truies soumises aux essais ont été placées dans des animaleries protégées, dix semaines avant la date présumée de mise-bas.

Les truies ont été inoculées 7 ou 8 semaines avant la mise-bas, par voie sous-cutanée; un rappel a été effectué 2 semaines avant la mise-bas, par voie intramusculaire. Chaque inoculum contenait 2 ml de suspension membranaire, soit 5 mg de protéines.

Deux séries d'essais ont été réalisés: 1ère série d'essais:
- une truie (10 043) a reçu le vaccin I,
- une truie (10 044) a reçu le vaccin II,
- deux truies (témoins 071 et 1010) n'ont pas été vaccinées.

2ème série d'essais:
- deux truies (20 008 et 20 012) ont reçu le vaccin II,
- une truie (20 013) a reçu le vaccin III,
- deux truies (témoins 20 005 et 20 007) n'ont pas été vaccinées.

Ces truies ont donné naissance aux nombres respectifs de porcelets indiqués dans le tableau suivant:

| Référence de la truie | Nombre de porcelets |
|---|---|
| 10 043 | 9 |
| 10 044 | 6 |
| 071 | 11 |
| 1010 | 6 |
| 20008 | 11 |
| 20012 | 4 |
| 20013 | 10 |
| 20005 | 9 |
| 20007 | 9 |

Tous les porcelets ont reçu à 2, 3, 4 et 5 jours d'âge, à raison de 0,5 ml par narine, une suspension de M. hyopneumoniae titrant $10^8$ UFC/ml (UFC = Unités formant colonies). La souche utilisée était la même que celle ayant servi à la préparation du vaccin.

Les porcelets ont été examinés chaque jour. La température rectale a été relevée, les signes cliniques ont été notés quotidiennement et des ponctions sanguines effectuées chaque semaine chez tous les porcelets. Ils ont été pesés et leurs consommation alimentaire a été évaluée chaque semaine.

Les porcelets ont été sacrifiés de façon échelonnée dans chaque groupe, de 5 à 10 semaines après l'infection. Après l'observation macroscopique des poumons, des contrôles bactériologiques et mycoplasmiques ainsi que des examens histologiques ont été entrepris. Les anticorps sériques ont été détectés par hémagglutination passive [M. Kobisch et Coll., Rec. Méd. Vét., 154 (1978), p. 847–852].

III–Résultats

1) Etat des porcelets issus des truies non traitées

Les résultats de la première série d'essais ont confirmé des observations précédentes: l'infection expérimentale par M. hyopneumoniae s'est traduite sur le plan clinique par de l'hyperthermie et de la toux apparaissant dans les jours qui ont suivi l'infection et se poursuivant durant la période d'induction des lésions pulmonaires. M. hyopneumoniae a exercé un effet dépressif sur la croissance des porcelets (par rapport aux animaux issus de truies vaccinées). Les lésions pulmonaires se sont montrées très étendues au moment des premiers sacrifices (5 semaines après l'infection) et jusqu'à la fin de l'expérience (10 semaines après l'infection). Contrairement à ce qui a été observé dans des expériences précédentes, il n'a pas été noté de sillons cicatriciels correspondant à des zones d'atélectasie indiquant l'évolution vers la cicatrisation. Ainsi, dans cette expérience, l'apparition des anticorps sériques décelés par hémagglutination passive a précédé la régression des lésions. Au plan anatomo-pathologique, l'existence d'une pneumonie caractérisée par des infiltrats lymphoïdes a confirmé les observations macroscopiques.

Les résultats de la 2ème série d'essais ont été moins tranchés. Le pouvoir pathogène de M. hyopneumoniae s'est exprimé moins nettement, en particulier chez les porcelets de la truie 20007, qui a eu des problèmes de santé au moment de la mise-bas et a reçu des antibiotiques aux jours J3 à J6 après la mise-bas. M. hyopneumoniae a induit de la toux chez les animaux infectés, mais a eu peu d'effet sur la croissance et la température corporelle. Les lésions pulmonaires ont été inconstantes chez les porcelets de la truie 20007. Il ne semble pas que M. hyopneumoniae se soit multiplié dans les poumons des porcelets de cette portée avant 60 jours alors qu'il était présent au niveau de la trachée dès les premiers sacrifices. M. hyopneumoniae a été retrouvé dans les poumons de 66% des porcelets de la truie 20005 (dans le mucus trachéal de 88%) et dans les poumons de 55% des porcelets de la truie 20007 (dans le mucus trachéal de 100%). Ces résultats montrent que les recherches microbiologiques de M. hyopneumoniae doivent être conduites à partir des poumons mais aussi du mucus trachéal qui se révèle être un bon témoin de la présence du mycoplasme.

Le taux des anticorps sériques décelés chez les porcelets n'a pas atteint le seuil de signification à la fin de l'expérience. Dans le cas des porcelets de la truie 20005, il faut noter que des anticorps colostraux ont été transmis, ce qui vraisemblablement a eu une incidence sur la synthèse des anticorps actifs faisant habituellement suite à l'infection. La multiplication réduite de M. hyopneumoniae dans les premiers stades de l'expérience explique le faible taux des anticorps chez les porcelets de la truie 20007.

2) Evaluation du potentiel vaccinant des membranes

Dans le modèle expérimental utilisé, l'effet protecteur du vaccin I (membrane + hydroxyde d'alumine) sur les porcelets a été de 100%. Dans le cas du vaccin II (membranes + agarose), il a été de 100% dans la première série d'essais et de 87% dans le deuxième série d'essais. L'efficacité du vaccin III (membranes précipitées par des anticorps) n'a été que de 70% (en écartant la pleuresie qui ne correspond pas aux lésions décrites lors d'une infection par M. hyopneumoniae).

On notera que les vaccins utilisés induisent des anticorps sériques chez les truies. Les porcelets issus de ces truies possèdent, après la prise colostrale, des anticorps d'origine maternelle dont le taux diminue rapidement en fonction du temps et atteint une valeur moyenne se situant au dessous du seuil de signification dans les semaines qui suivent la naissance. Six semaines après l'infection des porcelets, les truies présentent une remontée du taux des anticorps circulants. Enfin, M. hyopneumoniae est peu retrouvé chez les porcelets issus de truies vaccinées (13 à 26%).

3) Conclusion

Les expériences réalisées montrent que les membranes de M. hyopneumoniae, injectées par voies sous-cutanée et intra-musculaire à des truies gestantes, ont un effet protecteur significatif à l'égard des porcelets (absence de pneumonie et de retard de croissance). Ceci est particulièrement vrai dans le cas où de l'hydroxyde d'aluminium ou de l'agarose est utilisé comme adjuvant.

D'une part, les anticorps d'origine maternelle ayant une durée de vie de l'ordre de 3 à 4 semaines et d'autre part les animaux étant très sensibles à l'infection entre la 8e et la 12e semaine, il pourra dans certains cas être avantageux d'effectuer une vaccination complémentaire des porcelets à l'âge de 6 semaines, c'est-à-dire au moment où les anticorps passifs ont disparu et n'empêchent pas une prise vaccinale, avec un rappel de vaccination 3 semaines plus tard, c'est-à-dire à 9 semaines d'âge.

Par ailleurs, dans une expérience préliminaire, on a vacciné des porcs âgés de 12 semaines avec rappel de vaccination à 14 semaines et on a pu établir que les animaux réagissent à la vaccination en synthétisant des anticorps sériques (étude par hémagglutination passive).

Ces résultats montrent que le vaccin selon l'invention permet, avec un coût très réduit en raison de la simplicité de sa préparation, de lutter efficacement contre les maladies provoquées par les microorganismes ayant pour enveloppe la plus externe une membrane, notamment les mycoplasmes, et en particulier contre les maladies respiratoires qui affectent bon nombre d'élevages de porcins et diminuent leur rendement de façon importante.

Il est rappelé que le milieu de FRIIS a pour composition:

|   |   |   |
|---|---|---|
| | solution saline de HANKS | 500 ml |
| | eau distillée | 750 ml |
| A- | «Bacto brain heart infusion» (Difco) | 8,2 g |
| | «Bacto PPLO broth» (Difco) | 8,7 g |
| | extrait de levure | 60 ml |
| | rouge de phénol à 1% | 4 ml |
| B- | bacitracine | 500 mg (50 000 unités) |
| | acétate de thallium à 5% (MERCK) | 3 m |
| | sérum de cheval | 320 ml |

et que la solution saline de HANKS a pour composition:

|   |   |   |
|---|---|---|
| | NaCl | 80 g |
| | K cl | 4 g |
| solution A | Mg SO$_4$, 7H$_2$O | 1 g |
| | Mg Cl$_2$, 6H$_2$O dissolution dans 400 ml d'eau distillée | 1 g |
| | Ca C1$_2$ anhydre | 1,4 g |
| | eau distillée qsp | 500 ml |
| solution B | Na$_2$HPO$_4$, 12H$_2$O dissolution dans 400 ml d'eau distillée | 1,5 g |
| | KH$_2$PO$_4$ | 0,6 g |
| | eau distillée qsp | 500 ml |

## Revendications

1. Vaccin destiné à protéger contre les maladies provoquées par un mycoplasme, caractérisé en ce qu'il contient en tant que principe actif, des membranes plasmiques du microorganisme concerné, débarrassées, au moins dans une large mesure, des autres constituants dudit microorganisme.

2. Vaccin selon la revendication 1, caractérisé en ce qu'il se présente sous forme d'une suspension desdites membranes dans une solution de NaCl à 8,5 g/l, en présence d'un adjuvant qui assure le relargage progressif de l'immunogène.

3. Vaccin selon la revendication 2, caractérisé en ce que l'adjuvant est constitué par de l'hydroxyde d'aluminium, de l'agarose ou des anticorps anti-membrane du microorganisme concerné.

4. Vaccin selon l'une des revendications 1 à 3, caractérisé en ce que la concentration en protéines est située dans la gamme de 2 à 3 mg/ml.

5. Vaccin destiné à protéger les porcins contre la pneumonie enzootique, caractérisé en ce qu'il est essentiellement constitué par une suspension de membranes de Mycoplasma hyopneumoniae débarrassées au moins dans une large mesure, des autres constituants du microorganisme, correspondant à un poids de protéines de 2 à 3 g/l, dans une solution aqueuse de NaCl à 8,5 g/l en présence de 12 à 13 g/l d'hydroxyde d'aluminium en tant qu'adjuvant.

6. Vaccin destiné à protéger les porcins contre la pneumonie enzootique, caractérisé en ce qu'il est essentiellement constitué par une suspension de membranes de Mycoplasma hyopneumoniae débarrassées, au moins dans une large mesure, des autres constituants du microorganisme, correspondant à un poids de protéines de 2 à 3 g/l dans une solution aqueuse de NaCl à 8,5 g/l, en présence de 1,5 à 2 g/l d'agarose en tant qu'adjuvant.

7. Procédé pour la préparation du vaccin selon l'une des revendications 1 à 6, caractérisé en ce qu'il comprend essentiellement les étapes consistant à:

a) cultiver le mycoplasme contre lequel on désire préparer un vaccin jusqu'à la fin de la phase exponentielle de croissance;

b) récolter les cellules par centrifugation;

c) lyser les cellules récoltées par les ultrasons à 20 à 28 kHz, en plusieurs étapes, pendant 2 à 5 minutes au total;

d) sédimenter les membranes par centrifugation à environ 40 000 g pendant environ 1 heure;

e) les laver avec du tampon, centrifuger et disperser plusieurs fois; et

f) les introduire dans une forme utilisable pour la vaccination.

8. Suspension concentrée dans une solution aqueuse de NaCl à 8,5 g/l de membranes d'un mycoplasme, débarrassées, au moins dans une large mesure, des autres constituants du microorganisme pour l'utilisation en tant que principe actif d'un vaccin.

9. Membranes d'un mycoplasme, débarrassées, au moins dans une large mesure, des autres constituants du microorganisme, sous forme lyophilisée pour l'utilisation en tant que principe actif d'un vaccin.

10. Dose unitaire de vaccin ou inoculum destiné à protéger les porcins et les portées des truies gestantes contre les maladies provoquées par Mycoplasma hyopneumoniae, caractérisé en ce qu'il est essentiellement constitué par une suspension de membranes de Mycoplasma hyopneumoniae débarrassées, au moins dans une large mesure, des autres constituants du microorganisme et correspondant à un poids de protéines de 4 à 6 mg, de préférence 5 mg, dans 2 ml d'une solution aqueuse de NaCl à 0,85%, en présence de 1,20 à 1,30%, de préférence 1,25%, d'hydroxyde d'aluminium ou de 0,15 à 0,20%, de préférence 0,17%. d'agarose, ou encore d'une quantité saturante pour les membranes d'anticorps de porc antimembrane de Mycoplasma hyopneumoniae.

## Patentansprüche

1. Vakzin zum Schutz gegen Krankheiten, die durch ein Mycoplasma hervorgerufen werden, dadurch gekennzeichnet, dass es als Wirkstoff Plasmamembranen des betreffenden Mikroorganismus enthält, die, zumindest in hohem Masse, von anderen Bestandteilen dieses Mikroorganismus befreit sind.

2. Vakzin nach Anspruch 1, dadurch gekennzeichnet, dass es in der Form einer Suspension dieser Membranen in einer Natriumchloridlösung von 8,5 g/l in Gegenwart eines Zusatzstoffs, der die fortschreitende Aussalzung des Immunogens sichert, vorliegt.

3. Vakzin nach Anspruch 2, dadurch gekennzeichnet, dass der Zusatzstoff aus Aluminiumhydroxyd, Agarose oder aus gegen die Membran gerichteten Antikörpern des betreffenden Mikroorganismus besteht.

4. Vakzin nach einem der Ansprüche 1–3, dadurch gekennzeichnet, dass die Proteinkonzentration im Bereich von 2–3 mg/ml liegt.

5. Vakzin zum Schutz von Hausschweinen gegen enzootische Pneumonie, dadurch gekennzeichnet, dass es im wesentlichen aus einer Suspension von Membranen des Mycoplasma hyopneumoniae besteht, die, zumindest in hohem Masse, von anderen Bestandteilen des Mikroorganismus befreit sind, entsprechend einem Proteingewicht von 2 bis 3 g/l in einer wässrigen Lösung von 8,5 g/l Natriumchlorid in Gegenwart von 12 bis 13 g/l Aluminiumhydroxyd als Zusatzstoff.

6. Vakzin zum Schutz von Hausschweinen gegen enzootische Pneumonie, dadurch gekennzeichnet, dass es im wesentlichen aus einer Suspension von Membranen des Mycoplasma hyopneumoniae besteht, die, zumindest in hohem Masse, von anderen Bestandteilen des Mikroorganismus befreit sind, entsprechend einem Proteingewicht von 2 bis 3 g/l in einer wässrigen Lösung von 8,5 g/l Natriumchlorid in Gegenwart von 1,5 bis 2 g/l Agarose als Zusatzstoff.

7. Verfahren zur Herstellung des Vakzins nach einem der Ansprüche 1 bis 6, gekennzeichnet durch im wesentlichen folgende Schritte, bei denen

a) das Mycoplasma, gegen das ein Vakzin hergestellt werden soll, bis zum Ende der exponentiellen Wachstumsphase gezüchtet wird,

b) die Zellen durch Zentrifugieren geerntet werden,

c) die geernteten Zellen durch Ultraschall von 20 bis 28 kHz in mehreren Schritten während insgesamt 2 bis 5 Minuten lysiert werden,

d) die Membranen durch Zentrifugieren bei ungefähr 40 000 g während ungefähr 1 Stunde sedimentiert werden,

e) diese mit Puffer gewaschen, zentrifugiert und mehrere Male dispergiert werden, und

f) sie in eine für die Vakzination brauchbare Form überführt werden.

8. Konzentrierte Suspension von Membranen eines Mycoplasma, die, zumindest in hohem Masse von anderen Bestandteilen des Mikroorganismus befreit sind, in einer wässrigen Lösung von 8,5 g/l Natriumchlorid zum Gebrauch als Wirkstoff eines Vakzins.

9. Membranen eines Mycoplasma, die, zumindest in hohem Masse von anderen Bestandteilen des Mikroorganismus befreit sind, in lyophilisierter Form zum Gebrauch als Wirkstoff eines Vakzins.

10. Einzeldosis eines Vakzins oder Inoculums zum Schutz von Hausschweinen und der Würfe von trächtigen Mutterschweinen gegen Krankheiten, die durch Mycoplasma hyopneumoniae hervorgerufen werden, dadurch gekennzeichnet, dass sie im wesentlichen aus einer Suspension von Membranen des Mycoplasma hyopneumoniae besteht, die, zumindest in hohem Masse, von anderen Bestandteilen des Mikroorganismus befreit sind, und einem Proteingewicht von 4 bis 6 mg, vorzugsweise 5 mg, entsprechen, in 2 ml einer wässrigen 0,85%igen Natriumchloridlösung, in Gegenwart von 1,20 bis 1,30%, vorzugsweise 1,25%, Aluminiumhydroxyd, oder von 0,15 bis 0,20%, vorzugsweise 0,17% Agarose, oder ferner einer für die Membranen sättigenden Menge von gegen die Membranen gerichteten Schweine-Antikörpern von Mycoplasma hyopneumoniae.

**Claims**

1. Vaccine for protecting against diseases caused by a mycoplasm, characterized in that it contains as active principle, plasmic membranes of the micro-organism concerned, freed, at least to a large extent, from the other constituents of said micro-organism.

2. Vaccine according to claim 1, characterized in that it is in the form of a suspension of said membranes in a NaC1 solution at 8,5 g/l, in the presence of an adjuvant which ensures the progressive release of the immunogen.

3. Vaccine according to claim 2, characterized in that the adjuvant is constituted by aluminium hydroxide, agarose or antibodies against the membranes of the micro-organism conce.

4. Vaccine according to one of claims 1 to 3, characterized in that the protein concentration is within the range of 2 to 3 mg/ml.

5. Vaccine for protecting pigs against enzootic pneumonia, characterized in that it is essentially constituted by a suspension of membranes of Mycoplasma hyopneumoniae, freed, at least to a large extent, from the other constituents of the micro-organism, corresponding to a protein weight of 2 to 3 g/l, in an aqueous NaCl solution at 8,5 g/l in the presence of 12 to 13 g/l of aluminium hydroxid as adjuvant.

6. Vaccine for protecting pigs against enzootic pneumonia, characterized in that it is essentially constituted by a suspension of membranes of Mycoplasma hyopneumoniae, freed, at least to a large extent, from the other constituents of the micro-organism, corresponding to a protein weight of 2 to 3 g/l in an aqueous solution of NaCl at 8,5 g/l, in the presence of 1,5 to 2 g/l of agarose as adjuvant.

7. Process for the preparation of the vaccine according to any one of claims 1 to 6, characterized in that it essentially comprises the steps consisting of:

a) culturing the mycoplasm against which it is desired to prepare a vaccine up to the end of the exponential growth phase;

b) harvesting the cells by centrifugation;

c) lysing the harvested cells by ultrasounds at 20 to 28 kHz, in several steps, during 2 to 5 minutes on the whole;

d) sedimenting the membranes by centrifugation at about 40 000 g during about one hour;

e) washing them with a puffer, centrifugating and dispersing them several times; and

f) formulating them in a form useful for vaccination.

8. A concentrated suspension in an aqueous solution of NaCl at 8,5 g/l of membranes of a mycoplasm, freed, at least to a large extent, from the other constituents of the microorganism, for use as an active principle of a vaccine.

9. Membranes of a mycoplasm, freed, at least to a large extent, from the other constituents of the micro-organism, in freeze-dried form for use as an active principle of a vaccine.

10. A unit dose of vaccine or inoculum for protecting pigs and the unborn litters of pregnant sows against diseases caused by Mycoplasma hyopneumoniae, characterized in that it is essentially constituted by a suspension of membranes of Mycoplasma hyopneumoniae, freed, at least to a large extent, from the other constituents of the micro-organism and corresponding to a weight of proteins of 4 to 6 mg, preferably 5 mg, in 2 ml of an aqueous solution of NaC1 at 0,85%, in the presend of 1,20 to 1,30%, preferably 1,25%, of aluminium hydroxide or of 0,15 to 0,20%, preferably 0,17%, of agarose, or also of an amount saturating for the membranes, of pig antibodies against Mycoplasma hyopneumoniae membranes.